# EUROPEAN PATENT SPECIFICATION

(11) **EP 2 167 026 B1**
(45) Date of publication and mention of the grant of the patent: **09.12.2015**
(21) Application number: 08760661.2
(22) Date of filing: 06.06.2008
(51) Int. Cl.: A61K 8/49, A61Q 5/10

(54) **COSMETIC COMPOSITION FOR TREATMENT OF CANITIES**
KOSMETISCHE ZUSAMMENSETZUNG ZUR BEHANDLUNG VON CANITIES
COMPOSITION COSMÉTIQUE DESTINÉE AU TRAITEMENT DE LA CANITIE

(30) Priority: 11.06.2007 EP 07425366
(43) Date of publication of application: 31.03.2010
(73) Proprietor: Giuliani S.p.A., 20129 Milano (IT)
(72) Inventor: GIULIANI, Giammaria, I-20123 Milano (IT); BENEDUSI, Anna, I-20124 Milano (IT)
(74) Representative: Appoloni, Romano
(86) International application number: PCT/EP2008/057091
(87) International publication number: WO 2008/151994

(56) References cited:
- EP-A- 0 680 761
- EP-A- 0 693 278
- WO-A-00/30682
- FR-A- 2 574 660
- US-A1- 2004 015 214
- US-A1- 2005 000 040
- US-B1- 6 187 325
- DELEEUW JAAP ET AL.: "A case study to evaluate the treatment of vitiligo with khellin encapsulated in L-phenylalanin stabilized phosphatidylcholine liposomes in combination with ultraviolet therapy" EUROPEAN JOURNAL OF DERMATOLOGY, vol. 13, no. 5, September 2003 (2003-09), pages 474-477, XP009093121 France ISSN: 1167-1122
- CARLIE G ET AL: "KUVA stimulates proliferation and melanogenesis in normal huam melanocytes and melanoma cells in vitro" BRITISH JOURNAL OF DERMATOLOGY, vol. 149, 2003, pages 707-717, XP002461034

## Description

The present invention concerns the topical use of Khellin to reduce the amount of grey and white hair due to canities.

Khellin, or 4,9-dimethoxy-7-methyl-5H-furo[3,2-g][1]benzopyran-5-one is a derivative of coumarin which is known for its vasodilatory properties.

US4412071 (UpJohn) describes khellin analogues indicated as anti-arteriosclerosis agents.

EP680761 (Indena) relates to topical formulations based on khellin for cicatrizing the skin by means of its peripheral vasokinetic activity.

EP693278 (Indena) relates to formulations against hair loss based on khellin, combined with unsaturated fatty acids, again utilizing its peripheral vasokinetic activity.

Khellin has also been studied for therapy of the skin disorder known as vitiligo, characterized by non-pigmented skin patches. In this regard, research has shown how the use of NB (narrow band) UVB is particularly effective in the treatment of said disease. Procaccini (Ann. Ital. Dermatol. Clin. Sper., 1993, 47, 4) reports that applying khellin topically would not in itself have a specific repigmentation action against vitiligo, any effective repigmentation action being essentially due to induction by UV rays.

According to the present invention, it has been surprisingly found that khellin for topical use is effective in reducing the amount of white hair due to canities without having to combine it with other agents such as UV light.

Principally to achieve this activity but also other advantages which will be described henceforth, the current invention presents a composition for cosmetic use able to reduce the amount of white hair due to canities, characterized by comprising khellin as the active principle and excipients that render it suitable for topical use on hair.

According to the present invention, khellin can be present as the only active principle in the composition or in combination with one or more additional active principles, chosen from phenylalanine, ornithine, catechin, quercetin, copper salts such as copper gluconate.

The composition of the invention can be prepared in the form of a possibly liposomal hair treatment mask, or shampoo, or lotion, or conditioner, or mousse. Preferably, in a composition according to the invention khellin is comprised in a quantity between 0.2 and 1.8% w/v. With the aim of better understanding the characteristics of the invention, nonlimiting examples of its practical implementation are described in the following.

The quantity of each of the components is expressed as percentage weight per volume, % w/v, or, in the case of lotions, as weight in grams per 100 ml.

In the examples, where indicated, the INCI names of the components are given in accordance with the International Nomenclature of Cosmetic Ingredients.

### Example 1 - Liposomal hair mask

| | **Components** | **% w/v** |
|---|---|---|
| 1 | Glycerin | 3.00 |
| 5 | Tween 20 | 1.00 |
| 6 | Cremophor | 0.30 |
| 7 | Aristoflex | 0.70 |
| 8 | Phenonip | 0.98 |
| 9 | Copper gluconate | 0.10 |
| 11 | Cromollient SCE | 3.00 |
| 13 | Cremophor | 0.60 |
| 14 | Khellin | 1.0-1.5 |
| 15 | Phosal 75 SA | 3.00 |
| 16 | Parfum | 0.60 |
| 17 | Patent blue (0.2% solution) | 0.30 |
| 18 | Demineralized water | q.s. to 100 ml |

### Example 2 - Hair mask

| | **Components** | **% w/v** |
|---|---|---|
| 1 | Corn starch | 1.5 |
| 2 | BC 2366 | 2.5 |
| 3 | Disodium EDTA | 0.2 |
| 4 | Cetrimonium chloride | 4 |
| 5 | Salcare SC 92 | 2 |
| 6 | Glycerin | 4 |
| 7 | Nexbase 2004 | 2 |
| 8 | Fancol VB | 1.5 |
| 9 | BC 2262 | 0.5 |
| 10 | Lanette O | 1.5 |
| 11 | Palm oil | 0.8 |
| 12 | Brij 721 | 1 |
| 13 | Khellin | 0.5-1.5 |
| 14 | Crodazosoft DBQ | 8 |
| 15 | Cremophor RH 40 | 0.2 |
| 16 | Tween 20 | 0.9 |
| 17 | Glycerin | 3.0 |
| 18 | Incroquat behenyl tms | 0.5-0.7 |
| 19 | Cromollient SCE | 0.6 |
| 20 | Tween 20 | 0.1 |
| 21 | Parfum | 0.5 |
| 22 | Phenonip | 1 |
| 23 | Water | q.s. to 100 ml |

### Example 3 - Shampoo

| | **Components** | **% w/v** |
|---|---|---|
| 1 | EDTA | 0.20 |
| 2 | Polyquaternium-10 | 0.40 |
| 3 | Sodium Lauroyl Sarcosinate | 9.00 |
| 4 | Magnesium laureth sulfate (75%) and Disodium laureth sulfosuccinate (25%) | 38.00 |
| 5 | Potassium Undecylenoyl Hydrolyzed wheat protein | 1.00 |
| 6 | PEG-40 Hydrogenated Castor Oil | 0.50 |
| 7 | Polysorbate 20 | 1.00 |
| 8 | Cremophor RH 40 | 0.2 |
| 9 | Tween 20 | 0.9 |
| 10 | Glycerin | 3.0 |
| 11 | cromollient SCE | 0.6 |
| 12 | Prolipid 151 | 0.6 |
| 13 | khellin | 0.5-1.5 |
| 14 | tween 20 | 0.3 |
| 15 | cremophor RH40 | 6 |
| 16 | Phenyltrimethicone & Silicone Quaternium-15 | 0.50 |
| 17 | Silicone quaternium-15 & C11-15 Pareth-5 & C11-15 Pareth-9 | 0.80 |
| 18 | Parfum 12144 | 0.60 |
| 19 | BHA | 0.01 |
| 20 | Crodasilk liquid | 1.00 |
| 21 | Lauryl Methyl Gluceth-10 Hydroxypropyldimonium Chloride | 0.50 |
| 22 | Wheat (triticum vulgare) gliadins | 1.00 |
| 23 | Dore Gold AS232 | 0.05 |
| 24 | Glycol Distearate, Laureth-7, Sodium Cocoamphoacetate, Cocamidopropyl Betaine and Sodium Laureth Sulfate | 6.00 |
| 25 | PEG-90 Glyceryl Isostearate (and) Laureth-2 | 1.20 |
| 26 | PEG-120 Methyl Glucose dioleate | 1.20 |
| 27 | KCl | 0.60 |
| 28 | phenoxyethanol, methylparaben, butylparaben, ethylparaben, propylparaben | 0.98 |
| 29 | citric acid | 0.50 |
| 30 | Demineralized water | q.s. to 100.00 |

### Example 4 - Hair lotion

| | **INGREDIENTS (INCI names)** | **g in 100 ml** |
|---|---|---|
| 1 | Citric acid | 0.04-0.052 |
| 2 | Disodium EDTA | 0.03-0.06 |
| 3 | Alcohol | 15-20 |
| 4 | PEG-40 Hydrogenated castor oil | 0.5-3 |
| 5 | Tween 20 | 0.5-1.0 |
| 6 | Parfum | 0.10 |
| 7 | Khellin | 0.5-1.8 |
| 8 | Water | q.s. to 100 ml |

### Example 5 - Specific shampoo for problems of the scalp

| | **INGREDIENTS (INCI names)** | **% w/v** |
|---|---|---|
| 1 | Disodium EDTA | 0.10-0.30 |
| 2 | Polyquaternium-10 | 0.10-0.30 |
| 3 | Polyquaternium-55 | 0.1-0.50 |
| 4 | Citric acid | 0.60-0.80 |
| 5 | Potassium undecylenoyl hydrolyzed wheat protein | 3.6000 |
| 6 | Sodium lauroyl sarcosinate | 1.5000 |
| 7 | Cocamide mipa | 1.0-3.0 |
| 8 | Disodium laureth sulfosuccinate | 1.0-5.0 |
| 9 | Disodium capryloyl glutamate | 0.4-1.0 |
| 10 | Zinc coceth sulfate | 1.0-8.0 |
| 11 | PEG-200 hydrogenated glyceryl palmate | 0.5-3.0 |
| 12 | PEG-7 glyceryl cocoate | 0.5-2.0 |
| 13 | Laureth-2 | 0.05-2.0 |
| 14 | PEG-40 hydrogenated castor oil | 0.2-1.0 |
| 15 | Parfum | q.s. |
| 16 | Phenyl trimethicone | 0.05-3.0 |
| 17 | Silicone quaternium-15 | 0.005-1.0 |
| 18 | Laureth-4 | 0.010-0.5 |
| 19 | Khellin | 0.5-1.8 |
| 20 | Water | q.s. to 100 ml |

### Example 6 - Hair mousse

| | **Ingredients (INCI names)** | **% w/v** |
|---|---|---|
| 1 | Alcohol denat. | 12.0-15.0 |
| 4 | Ethoxydiglycol | 1.0-3.0 |
| 5 | Sodium lauryl sulfate | 0.5-2.0 |
| 6 | Sodium hydroxide | 0.01-0.06 |
| 7 | Citric acid | 0.01-0.06 |
| 8 | Peg-40 hydrogenated castor oil | 0.30-0.60 |
| 9 | Parfum | q.s |
| 10 | Tocotrienols | 0.01-0.05 |
| 11 | Potassium undecylenoyl hydrolyzed wheat protein | 0.050-1.0 |
| 12 | Zinc coceth sulfate | 0.050-0.1 |
| 13 | Polyquaternium-16 | 0.020 |
| 14 | Khellin | 0.2-0.5 |
| 15 | Water | q.s. to 100ml |

### Example 7 - Liposomal mask for hair

| | **Components** | **% w**/**v** |
|---|---|---|
| 1 | Glycerin | 3.00 |
| | Ornithine | 0.20-0.9 |
| 3 | Phenylalanine | 0.20-0.60 |
| 4 | Pycnogenol | 0.10-0.50 |
| 5 | Tween 20 | 1.00 |
| 6 | Cremophor | 0.30 |
| 7 | Aristoflex | 0.70 |
| 8 | Phenonip | 0.98 |
| 9 | Copper gluconate | 0.10 |
| 10 | Catechin | 0.05-0.1 |
| 11 | Cromollient SCE | 3.00 |
| 12 | Quercetin | 0.01-0.1 |
| 13 | Cremophor | 0.60 |
| 14 | Khellin | 0.3-1.3 |
| 15 | Phosal 75 SA | 3.00 |
| 16 | Parfum | 0.60 |
| 17 | Patent blue (0.2% solution) | 0.30 |
| 18 | Demineralized water | q.s. to 100 ml |

### Example 8 - Hair mask

| | **Components** | **% w/v** |
|---|---|---|
| 1 | Corn starch | 1.5 |
| 2 | Ornithine | 0.8-1.20 |
| 3 | Phenylalanine | 0.1-0.40 |
| 4 | Copper gluconate | 0.05-0.1 |
| 5 | BC 2366 | 2.5 |
| 6 | Disodium EDTA | 0.2 |
| 7 | Cetrimonium chloride | 4 |
| 8 | Salcare SC 92 | 2 |
| 9 | Glycerin | 4 |
| 10 | Nexbase 2004 | 2 |
| 11 | Ceramide A2 | 0.2-0.5 |
| 12 | Fancol VB | 1.5 |
| 13 | BC 2262 | 0.5 |
| 14 | Lanette O | 1.5 |
| 15 | Palm Oil | 0.8 |
| 16 | Brij 721 | 1 |
| 17 | Khellin | 0.5-1.5 |
| 18 | Crodazosoft DBQ | 8 |
| 19 | Catechin | 0.025-0.07 |
| 20 | Cremophor RH 40 | 0.2 |
| 21 | Tween 20 | 0.9 |
| 22 | Glycerin | 3.0 |
| 23 | Panthenol | 0.1-0.25 |
| 24 | Nanocream | 0.8-1.2 |
| 25 | Incroquat behenyl tms | 0.5-0.7 |
| 26 | Quercetin | 0.01-0.005 |
| 27 | Cromollient SCE | 0.6 |
| 28 | Tween 20 | 0.1 |
| 29 | Parfum | 0.5 |
| 30 | Phenonip | 1 |
| 31 | Water | q.s. to 100 ml |

### Example 9 - Shampoo

| | **Components** | **% w/v** |
|---|---|---|
| 1 | EDTA | 0.20 |
| 2 | Ornithine | 0.5-1.2 |
| 3 | Phenylalanine | 0.2-0.6 |
| 4 | Copper gluconate | 0.08-0.12 |
| 5 | Polyquaternium-10 | 0.40 |
| 6 | Sodium Lauroyl Sarcosinate | 9.00 |
| 7 | Magnesium laureth sulfate (75%) and Disodium laureth sulfosuccinate (25%) | 38.00 |
| 8 | Potassium Undecylenoyl Hydrolyzed wheat protein | 1.00 |
| 9 | PEG40 Hydrogenated Castor Oil | 0.50 |
| 10 | Polysorbate 20 | 1.00 |
| 11 | Catechin | 0.025-0.07 |
| 12 | Pycnogenol | 0.05-0.15 |
| 13 | Cremophor RH 40 | 0.2 |
| 14 | Tween 20 | 0.9 |
| 15 | glycerin | 3.0 |
| 16 | panthenol | 0.2 |
| 17 | nanocream | 10 |
| 18 | Quercetin | 0.01-0.005 |
| 19 | cromollient SCE | 0.6 |
| 20 | Prolipid 151 | 0.6 |
| 21 | khellin | 0.5-1.5 |
| 22 | tween 20 | 0.3 |
| 23 | cremophor RH40 | 6 |
| 24 | Phenyltrimethicone & Silicone Quaternium-15 | 0.50 |
| 25 | Silicone quaternium-15 & C11-15 Pareth-5 & C11-15 Pareth-9 | 0.80 |
| 26 | Parfum 12144 | 0.60 |
| 27 | BHA | 0.01 |
| 28 | Crodasilk liquid | 1.00 |
| 29 | Lauryl Methyl Gluceth-10 Hydroxypropyldimonium Chloride | 0.50 |
| 30 | Wheat (triticum vulgare) gliadins | 1.00 |
| 31 | Hairspheres Biogenina | 0.2-0.3 |
| 32 | Dore Gold AS 232 | 0.05 |
| 33 | Glycol Distearate, Laureth-7, Sodium Cocoamphoacetate, Cocamidopropyl Betaine and Sodium Laureth Sulfate | 6.00 |
| 34 | PEG-90 Glyceryl Isostearate (and) Laureth-2 | 1.20 |
| 35 | PEG-120 Methyl Glucose dioleate | 1.20 |
| 36 | KCI | 0.60 |
| 37 | phenoxyethanol, methylparaben, butylparaben, ethylparaben, propylparaben | 0.98 |
| 38 | citric acid | 0.50 |
| 39 | Demineralized water | q.s. to 100.00 ml |

### Example 10 - Hair lotion

| | **INGREDIENTS (INCI names)** | **g in 100 ml** |
|---|---|---|
| 1 | Citric acid | 0.04-0.052 |
| 2 | Disodium EDTA | 0.03-0.06 |
| 3 | Alcohol | 15-20 |
| 4 | PEG-40 Hydrogenated castor oil | 0.5-3 |
| 5 | Tween 20 | 0.5-1.0 |
| 6 | Parfum | 0.10 |
| 7 | Khellin | 0.5-1.8 |
| 8 | Ornithine | 0.2-0.5 |
| | Water | q.s. to 100 ml |

### Example 11 - Specific shampoo for problems of the scalp

| | **INGREDIENTS (INCI names)** | **% w/v** |
|---|---|---|
| 1 | Disodium EDTA | 0.10-0.30 |
| 2 | Polyquaternium-10 | 0.10-0.30 |
| 3 | Polyquaternium-55 | 0.1-0.50 |
| 4 | Citric acid | 0.60-0.80 |
| 5 | Ornithine | 0.10-0.05 |
| 6 | Potassium undecylenoyl hydrolyzed wheat protein | 3.6000 |
| 7 | Sodium lauroyl sarcosinate | 1.5000 |
| 8 | Cocamide mipa | 1.0-3.0 |
| 9 | Phenylalanine | 0.10-0.50 |
| 10 | Disodium laureth sulfosuccinate | 1.0-5.0 |
| 11 | Disodium capryloyl glutamate | 0.4-1.0 |
| 12 | Zinc coceth sulfate | 1.0-8.0 |
| 13 | Peg-200 hydrogenated glyceryl palmate | 0.5-3.0 |
| 14 | Peg-7 glyceryl cocoate | 0.5-2.0 |
| 15 | Laureth-2 | 0.05-2.0 |
| 16 | Peg-40 hydrogenated castor oil | 0.2-1.0 |
| 17 | Parfum | q.s. |
| 18 | Phenyl trimethicone | 0.05-3.0 |
| 19 | Silicone quaternium-15 | 0.005-1.0 |
| 20 | Laureth-4 | 0.010-0.5 |
| 21 | Khellin | 0.5-1.8 |
| 22 | Catechin | 0.025-0.07 |
| 23 | Quercetin | 0.01-0.005 |
| 24 | Water | q.s. to 100 ml |

### Example 12 - Hair mousse

| | **Ingredients (INCI names)** | **% w/v** |
|---|---|---|
| 1 | Alcohol denat. | 12.0-15.0 |
| 2 | Ornithine | 0.050-0.20 |
| 3 | Salicylic acid | 0.05-0.02 |
| 4 | Ethoxydiglycol | 1.0-3.0 |
| 6 | Sodium lauryl sulfate | 0.5-2.0 |
| 7 | Sodium hydroxide | 0.01-0.06 |
| 8 | Citric acid | 0.01-0.06 |
| 9 | Panthenol | 0.200 |
| 10 | Glutathione | 0.10-0.5 |
| 11 | P-hydroxyanisole | 0.05-0.12 |
| 12 | Potassium azeloyl diglycinate | 0.090 |
| 13 | Phytic acid | 0.01-0.03 |
| 14 | Peg-40 hydrogenated castor oil | 0.30-0.60 |
| 15 | Parfum | q.b |
| 16 | Tocotrienols | 0.01-0.05 |
| 17 | Potassium undecylenoyl hydrolyzed wheat protein | 0.050-1.0 |
| 18 | Zinc coceth sulfate | 0.050-0.1 |
| 19 | Polyquaternium-16 | 0.020 |
| 20 | Phenylalanine | 0.2-0.6 |
| 21 | Khellin | 0.2-0.5 |
| 22 | Water | q.s to 100 ml |

With the aim of better understanding the advantages of the invention, the following describes a clinical study carried out on healthy volunteers.

### CLINICAL STUDY

An assessment was made of the effectiveness of a topical treatment for reducing the amount of white hair due to canities of two compositions in accordance with the invention, i.e.
1) hair mask formulated with liposomes
2) shampoo formulated with liposomes

A double-blind clinical study was carried out using a mask 1-A in accordance with the invention comprising 1.5% w/v khellin in combination with phenylalanine, ornithine, copper gluconate and excipients suitable for topical application to hair, compared with the use of a placebo formulation 1-B, i.e. a similar mask base for hair but without the active principle.

50 volunteers, comprising 40 women and 10 men between 35 and 55 years of age, and healthy according to specific inclusion and exclusion criteria, including the exclusion of immunological pathologies and of systemic drug consumption were treated as follows.

1-A and 1-B were applied three times a week for eight weeks to each subject. At the end of the treatment, each subject was evaluated by:
- dermatological examination
- macro photograph (digital)
- epiluminescence (Molemax II)
- Reflectance Confocal Microscopy (RCM) using a Lucid confocal microscope.

In 65% of the subjects treated with mask 1-A a 20-30% reduction in grey/white hair was found compared with a 2% reduction in grey/white hair in subjects treated with mask 1-B, with reduction of up to a maximum of 5%, p = 0.05.

Additionally, of those treated with mask 1-A, an increase in the anagen phase of the hair growth cycle (compared to the baseline) as well as an increase in the hair shaft diameter were found in subjects affected by androgenetic alopecia.

After having completed the assessment on the treatment with mask 1), the subjects in active group A used a gentle shampoo 2-A containing 1% khellin and formulated with liposomes, to be used twice weekly for four weeks.

The subjects in non-active group B used a normal gentle shampoo 2-B as a comparison, i.e. without active principles.

At the end of the four weeks, the assessments were repeated on all subjects.
- in group A it was found that the results obtained with mask 1-A application were maintained.
- in group B with free shampoo 2-B a slight increase in grey hair was noted, but was not statistically significant compared to the baseline of the non-active group. In conclusion, application of mask 1-A with the active principle of the invention has demonstrated a capacity to increase the number of repigmented hairs even though repigmentation of the whole head of hair was not observed.

Application of shampoo 2-A was shown to be effective in maintaining the action of mask 1-A treatment.

No systemic or localized side effects were noted on use of the products at the end of the reported study.

The composition of the invention is mainly indicated for anti-ageing cosmetic use, for preventing and treating canities and hence ageing of the hair.

It is particularly indicated for the treatment of greying hair and hair in the initial stages of canities for reducing its progression.

Following topical application of the composition of the invention to hair, an increase in the anagen phase of the hair growth cycle (compared to the baseline) as well as an increase in hair shaft diameter in subjects affected by androgenetic alopecia was generally also apparent.

For this reason, the composition of the invention is particularly indicated for the treatment of canities associated with androgenetic alopecia.

## Claims

1. Use of khellin as a cosmetic for reducing the amount of grey or white hair due to canities.

2. Use of khellin for reducing the amount of grey or white hair due to canities, while at the same time producing an increase in the anagen phase and hair shaft diameter in subjects affected by androgenetic alopecia.

3. Use according to the preceding claims wherein khellin is formulated in a composition comprising excipients suitable for topical use on hair.

4. Use according to the preceding claims wherein khellin is used in combination with one or more active compounds chosen from: phenylalanine, ornithine, catechin, quercetin, copper salts.

5. Use according to the preceding claims wherein khellin is prepared in the form of a mask for hair treatment, or shampoo, or lotion, or conditioner, or mousse.

6. Use according to claim 5 wherein the said form contains liposomes.

7. Use according to the preceding claims wherein khellin is prepared in a composition in a quantity from 0.2 to 1.8% w/v.

## Patentansprüche

1. Verwendung von Khellin als kosmetisches Präparat zur Verminderung der Menge an grauem oder weißem Haar, welches auf Canaties zurückzuführen ist.

2. Verwendung von Khellin zur Verminderung der Menge an grauem oder weißem Haar, welches auf Canaties zurückzuführen ist, während gleichzeitig bei Personen, die an androgenetischem Haarausfall leiden, eine Zunahme in der Anagenphase und im Haarschaftdurchmesser erzeugt wird.

3. Verwendung nach den vorangehenden Ansprüchen, wobei Khellin in einer Zusammensetzung formuliert wird, welche Trägerstoffe umfasst, die für die äußerliche Anwendung am Haar geeignet sind.

4. Verwendung nach den vorangehenden Ansprüchen, wobei Khellin in Kombination mit einer oder mehreren aktiven Verbindungen benutzt wird, die unter den folgenden ausgewählt werden: Phenylalanin, Ornithin, Catechin, Quercetin, Kupfersalze.

5. Verwendung nach den vorangehenden Ansprüchen, wobei Khellin in Form einer Haarbehandlungsmaske oder als Shampoo oder Lotion oder Trägerstoff oder Schaum hergestellt wird.

6. Verwendung nach Anspruch 5, wobei die genannte Formulierung Liposome enthält.

7. Verwendung nach den vorangehenden Ansprüchen, wobei Khellin in einer Zusammensetzung hergestellt wird, die eine Menge von 0,2 bis 1,8 Gew.-% enthält.

## Revendications

1. Utilisation de la khelline en tant que cosmétique pour réduire la quantité de cheveux gris ou blancs dus à la canitie.

2. Utilisation de la khelline pour réduire la quantité de cheveux gris ou blancs dus à la canitie, tout en produisant en même temps une augmentation de la phase anagène et du diamètre de la tige du cheveu chez des sujets affectés par une alopécie androgénétique.

3. Utilisation selon les revendications précédentes, où la khelline est formulée dans une composition comprenant des excipients appropriés pour une utilisation topique sur les cheveux.

4. Utilisation selon les revendications précédentes, où la khelline est utilisée en combinaison avec un ou plusieurs composés actifs choisis parmi : la phénylalanine, l'ornithine, la cathéchine, la quercétine, les sels de cuivre.

5. Utilisation selon les revendications précédentes, où la khelline est préparée sous la forme d'un masque pour le traitement des cheveux, ou d'un shampooing, ou d'une lotion, ou d'un après-shampooing, ou d'une mousse.

6. Utilisation selon la revendication 5, où ladite forme contient des liposomes.

7. Utilisation selon les revendications précédentes, où la khelline est préparée dans une composition dans une quantité de 0,2 à 1,8 % p/v.
